(19) Europäisches Patentamt
European Patent Office
Office européen des brevets

(11) EP 1 759 703 A1

(12) EUROPEAN PATENT APPLICATION
published in accordance with Art. 158(3) EPC

(43) Date of publication:
07.03.2007 Bulletin 2007/10

(21) Application number: 04808945.2

(22) Date of filing: 24.06.2004

(51) Int Cl.:
*A61K 31/785* (2006.01) *A61P 31/00* (2006.01)

(86) International application number:
PCT/RU2004/000242

(87) International publication number:
WO 2006/011823 (02.02.2006 Gazette 2006/05)

(84) Designated Contracting States:
AT BE BG CH CY CZ DE DK EE ES FI FR GB GR HU IE IT LI LU MC NL PL PT RO SE SI SK TR

(71) Applicant: Akciju Sabiedriba "Olainfarm"
2114 Olaine (LV)

(72) Inventors:
- MARCHUKOV, Valery Aleksandrovich
St. Petersburg, 193076 (RU)
- SELKOV, Sergei Alekseevich
St. Petersburg, 197198 (RU)
- CHERNOBROVY, Aleksandr Nikolaevich
LV-2114 Olaine (LV)
- SELJUTIN, Aleksandr Vasilievich
St. Petersburg, 195196 (RU)
- SOKOLOV, Dmitry Igorevich
St.Petersburg, 195269 (RU)

(74) Representative: Zellentin, Rüdiger et al
Patentanwälte
Zellentin & Partner
Zweibrückenstrasse 15
80331 München (DE)

# (54) IMMUNISTIMULATIVE AND INTERFERONOGENOUS AGENT

(57) The invention relates to the area of biology and medicine, and specifically to new biologically active substances. Object of the invention is to produce new immunostimulating and interferonogenic agents. This task is solved by the use of salts of 3.5-dimethyl-1-adamantilammonuim and water-soluble carboxylated (co)polymers of the general formula as immunostimulating and interferonogenic agents:

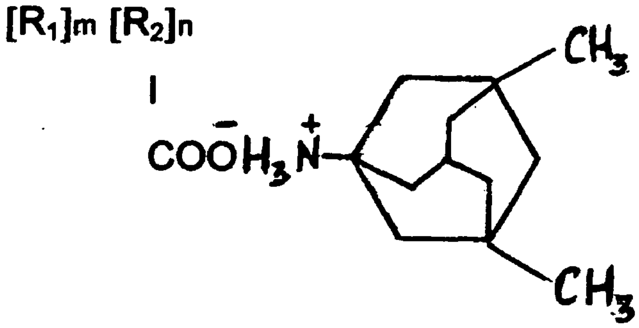

where m and n are mol%, m = 100 - n,
and carboxylated (co)polymers are selected from the group:
polyvinylaminosuccinic acid, a copolymer of vinyl alcohol and N-vinylaminosuccinic acid, a copolymer of N-vinylpyrrolidone and N-vinylaminosuccinic acid. The results of comparative analysis of the immunostimulating and interferonogenic activity and toxicity of compounds are given. They showed rather high claimed activity (higher than polyrem); at that, they compare favourably by the nature of their antiviral durable action, which allows using the said polymeric salts as immunostimulating and interferonogenic agents for sparing treatment and prevention of viral diseases. A general synthesis method and examples of each of the offered agents are described. Produced compounds are expressly identified by IR analysis, elements analysis, and potentiometric titration.

EP 1 759 703 A1

## Description

### Field of the invention

This invention relates to the fields of biology and medicine, to the production of new biologically active substances, and is intended for the use as immunostimulating and interferonogenic agents for medical, prophylactic and explorative purposes.

### Background of the invention

Many immunostimulating and interferongenic medications are known, they are described both in reference editions (e.g., see M.D. Mashkovski "Medicinal Agents", M, "New Wave". 2002; Yu.F. Krylov "Encyclopedia of Drugs/ Register of Medicinal Agents of Russia". M, 2000) and special literature. The most famous among them are Larifan, Ridostine, Kagocel, Rogaxin, Gozalidon, Savrats, Megacin, Amixine, Cycloferone, Neovir, Ampligen, Poludan, and Polyguatsil. From the drugs offered lately Polyrem - an antiviral agent possessing interferonogenic properties and a capacity to enhance the cytotoxic function of lymphocytes (natural killers) - is worth noting.

Many of them are extensively and effectively used in medical practice, e.g., Likopide and Cycloferone are used successfully in the therapy of a wide range of viral infections. At that, as all other medicinal agents, besides curative effect, immunostimulating and interferonogenic drugs have side effects different by force, direction, and duration. Moreover, long-term taking of one and the same drug leads to accustomitization, and its effectiveness decreases considerably with the time. That is why new medications, which are more effective, less toxic, and with longer and directional effect and fewer side effects are always searched for. This search is implemented in different classes of natural and synthetic compounds, including adamantine derivatives.

The drug memantine is known, it is hydrochloride 3.5-dimethyl-1-adamantylamine and is recommended for treatment of Parkinson's disease (M.D. Mashkovski "Medicinal Agents", in 2 volumes, M, "New Wave". 2002, vol.1, p. 143).

Midantan medication (hydrochloride 1-adamantylamine) is used for treatment of Parkinsonism. Besides, it shows antiviral effect against the virus of A type influenza of (ibid, vol.1, p.142).

Hydrochloride α-methyl-α-(1-adamantl)methylamine, which is a substance of the medication remantadine, is efficient against the virus of A type influenza of (ibid., vol.2, p.325), but there is no information as to its activity for treatment of Parkinsonism.

The above short review of biological properties of the amino derivatives of adamantan shows that even a slight structural change of these homotypes and isomers causes rather significant and complex changes of their properties which are unobvious for a specialist in advance.

The above listed medications are rather effective in the said usage directions but are quickly excreted from the organism. This requires their frequent intake and a dose increase which causes unfavourable side effects, and intoxication of the organism (ibid., pp. 142, 143, 325).

It is also known that the effectiveness of the therapeutic action of a physiologically active substance in the result of its adjoining to a polymer can change considerably. Production of the polymeric structure of a physiologically active substance may cause appearance of new properties, enhancement of the existing ones as well as loss of physiological activity. Thus, the antibacterial activity of the product of kanamycine interaction with dialdehyde dextran where an antibiotic is adjoined by a covalent bond is close to the activity of an initial kanamycine. At the same time ampicillin adjoining to dialdehyde dextran is accompanied by a considerable (10 - 15 times) decrease of this antibiotic activity. A different degree of decrease in the activity of kanamycine and ampicillin when they are adjoined to dialdehyde dextran depends on the influence of a polymer molecule on the electrolytic resistance of linking, even of one type (V.A. Snezhko et al. - Antibiotics, 1972, vol.17, №1, pp.48 - 52).

In a number of cases polymer analogs can lose their biological properties characteristic for low-molecular structures. Thus, e.g. poly-2-vinylpiridine-N-oxide and some other N-oxides are well known for their antisilicosis activity; at the same time polymers containing N-oxide groups screened by an alkylene chemical group do not feature this activity. (E.F. Razvodovski in col. Progress of Polymer Chemistry and Physics, M, Chemistry, 1973, p.305).

As the analysis of known and partially listed developments shows, search and exploration of new polymeric compounds in the row of amino derivatives of adamantan is prospective for significant enhancement of biological properties as compared to the initial low-molecular aminocompound, for extension of its efficacy time and expansion of a biological activity range. However, unfortunately, till now this search has been based not on an accurate theoretical analysis but on a researcher's intuition and numerous tests.

An unexpected and fortunate finding was polymeric compounds of adamantylamine (USSR, author's certificate №523618, Mcl. C08F 8/32, 1975) and polymeric salts of α-methyl-α-(1-adamantil) methylamine (USSR, author's certificate №640544, Mcl. C08F 220/04, C08F 8/32, 1978) which possessed a prolonged antiviral action against the virus of A type influenza. In particular, based on a vinyl alcohol copolymer with N-vinylaminosuccinic acid and α-methyl-α-(1-adamantil) methylamine their polymeric salt was produced; it is a substance of the medication polyrem. The medication polyrem was found to have not only a prolonged action against the virus of A type influenza, but also (different from polymeric compounds of adamantylamine) an antiviral activity against viruses of herpes of I and II types, and tick-borne encephalitis. It induces interferon and has an immunostimulating effect. The medication polyrem is mainly used for prevention and treatment of the said viral infections (patent of the RF №2071323, Mcl. 6 A61 K 31/785; A61K 9/06, 9/20 "Antiviral medication POLYREM" published on 10.01.1997; F.I. Ershov Antiviral Medications, M, Medicine, 1998, pp. 141- 142).

This medication is the closest to the claimed compounds by its purpose and chemical composition; it is selected as a prototype.

## Objects of the invention

It is an object of the invention to produce new polymeric compounds possessing an immunostimulating and interferonogenic effect.

## Task solution

The determined task is solved by using salts of 3.5-dimethyl-1-adamantylammonium and water-soluble carboxylated copolymers of a general formula

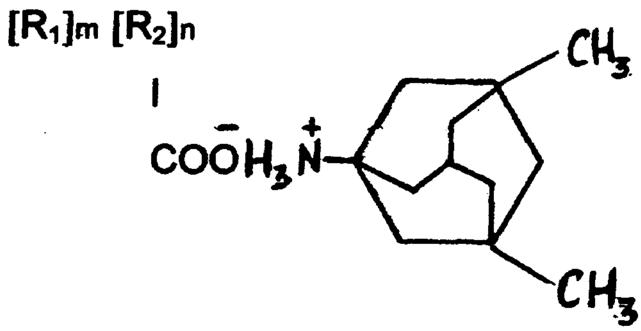

where m and n - mol %, m=100-n,
and carboxylated copolymers are selected from the group:
polyvinylaminosuccinic acid,
copolymer of vinyl alcohol and N-vinylaminosuccinic acid,
copolymer of N-vinylpyrrolidone and N-vinylaminosuccinic acid.

1) Polyvinylaminosuccinic acid (PVASA)

$R_1$ = - $CH_2$ – CH - ; $R_2$ = - $CH_2$ – CH-
| |
NH NH
| |
CO-$(CH_2)_2$ –COOH CO – $(CH_2)_2$ -

where n = 1- 100, better 5-100

2) Copolymer of vinyl alcohol and N-vinylaminosuccinic acid (VA)m - (VASA)n

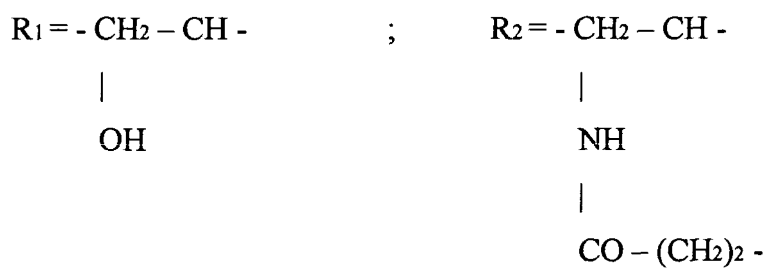

where n = 1 - 99, better 5 - 50

3) Copolymer of N-vinylpyrrolidone and N-vinylaminosuccinic acid (VP)m - (VASA)n

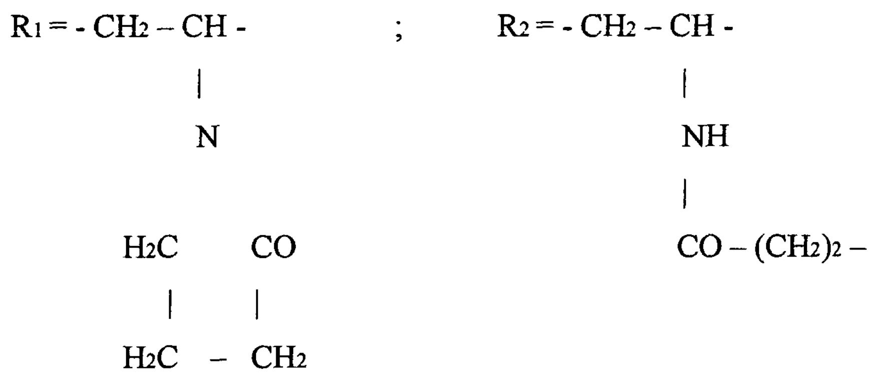

where 1 - 99, better 5 - 50

The possibility of production of neutral and acid salts of PVASA with claimed properties corresponds to the values n=1- 100.
In copolymers of (VA)m - (VASA)n and (VP)m - (VASA)n the values m and n in accordance with the reaction capacity of the monomers used in the polymer preparation method can be in the range n = 1 - 99 and m = 100 - n

The efficacy level of the immunostimulating and interferonogenic activity is achieved with $n \geq 5$ and the increase of n-values over 50 influences the activity level insignificantly. That is why to ensure claimed properties of polymeric salts the use of the value n = 5 - 50 is necessary and sufficient.

Offered salts were first announced in the international application PCT/RU2003/000417 dated September 23, 2003, WO 2005/028528 A1, published March 31, 2005. It gives full description of the structures of these salts, their synthesis, identification, as well as the results of biological properties study. The inventors managed to find in offered compounds only antiviral activity against the viruses of A and B types influenza. At that an important advantage of these substances is their prolonged effect allowing their use for sparing treatment and prevention of influenza. The application does not contain any assumptions concerning their possible immunostimulating and interferonogenic properties. These properties are in no way related to antiviral activity and do not follow explicitly from the current knowledge level in this area.

**Summary of the invention**

**(Detailed description of the invention)**

The essence of the invention is explained by the information stated further, and namely:

- general method of claimed salts synthesis;
- 3 examples of synthesis (for each of claimed salts);
- identification of produced compounds;
- determination of biological properties of synthesized salts (immunostimulating activity, interferonogenic activity and toxicity).

General method of claimed salts synthesis

The producing method for polymeric salts of 3.5-dimethyl-1-adamantylammonium is the interaction of a carboxylated (co)polymer with 3.5-dimethyl-1-adamantylamine in the aquatic medium at 15 - 25 C° with mixing of the components for 0.5 - 1 hour and introduction of equimolecular ratios of reagents as calculated per actual or given carboxyl groups content in a (co)polymer and further recovery of salts from the solution by precipitation or using one of the spray or sublimation drying types.

A general initial reagent in the synthesis of polymeric salts, 3.5-dimethyl-1-adamantylamine, can be produced and used as an intermediate product in the synthesis of its hydrochloride or can be isolated from commercial-grade hydrochloride (memantine) by its treatment with 5% water solution of sodium hydrate and further extraction by ethyl ether or chloroform. Then ether (chloroform) is distilled and 3.5-dimethyl-1-adamantylamine is distilled under vacuum. Produced 3.5-dimethyl-1-adamantylamine does not change its biological activity and is a colorless oily liquid, insoluble in water but soluble in dimethyl sulfoxide, as well as concentrated and weak acetic and hydrochloric acid.

The pureness of produced 3.5-dimethyl-1-adamantylamine was assessed by the data of the element analysis and infrared analysis. The results of the element analysis of $C_{12}N_{21}N$:

| | | | |
|---|---|---|---|
| Calculated, %: | C=80.37; | H=11.80; | N=7.81 |
| Found, %: | C=80.41; | H=11.95; | N=7.70 |

As the second component - a polymeric base - compositionally homogeneous (by their chemical composition and molecular weight) carboxylated, water soluble, and non-toxic (co)polymers were selected:

1) polyvinylaminosuccinic acid (PVASA);
2) copolymer of vinyl alcohol and N-vinylaminosuccinic acid (VA)m - (VASA)n;
3) copolymer of N-vinylpyrrolidone and N-vinylaminosuccinic acid (VP)m - (VASA)n.

The listed polymers can be produced according to the earlier described method (A.F. Nikolaev et al. The Journal of Applied Chemistry, 1970, vol..43, №6, pp. 1339 - 1343; A.F. Nikolaev et al. High-molecular Compounds, 1972, vol. 14A, №11, pp. 2368 - 2370).

Molecular weight of used polymers is in the range of 20 - 80kDa, the number of carboxylated links in co(polymers) is from 1 to 100 mol%.

Salt formation reaction of 3.5-dimethyl-1-adamantylamine with carboxylated polymers is implemented in the aquatic medium at the temperature of 15 - 25 C°, with mixing of the components for 0.5 - 1 hour and introduction of equimolecular ratios of reagents as calculated per actual (or rated) carboxyl groups content in a (co)polymer with further isolation of salts from the solution by precipitation into acetone or methyl-ethyl ketone, or the mixture of ethyl alcohol and diethyl ether, or diethyl ether and benzol, or using one of the spray or sublimation drying types. Produced compounds are polymeric salts of polycarboxilic acids and 3.5-dimethyl-1-adamantylamine (3.5-dimethyl-1-adamantylammonium - (DMAAM). The composition and structure of produced salts were identified based on the data of element analysis, infrared analysis, and potentiometric titration in non-aquatic medium.

Synthesis method and other examples are described in much detail in the international application PCT/RU2003/000417 dated 23.09.03 (WO 2005/028528 published March 31, 2005).

Synthesis examples of claimed saltsio

Example 1. Into a kettle (retort) equipped with a mixer, 0.858g of poly-N-vinylaminosuccinic acid (PVASA) and 30cm$^3$ of distilled water are loaded. After solution of the polymer, 1.074g of 3.5-dimethyl-1-adamantylamine (DMAAM) is introduced. The initial molecular ratio of VASA:DMAAM is 1:1. The reaction takes place at the temperature of 15 C° during 1 hour, after that 30m$^3$ of ethyl alcohol is poured into the reaction mixture and the product is produced by precipitation into the mixture of benzol and diethyl ether (ratio 1:3). Sediment is filtrated, irrigated with diethyl ether and dried at 30 - 35 C°/6.65Pa. Based on the results of the elements analysis and potentiometric titration DMAAM content in the produced salt is 54.32 wt% which corresponds to the links content of 3.5-dimethyl-1-adamantylamine-ammonium salt of vinylaminosuccinic acid (VASA - DMAAM) in the amount of 95 mol%. Product output (PVASA - DMAAM) was 89%. Characterizing viscosity of a polymeric salt solution in HCl concentration c(HCl) 0. 1mol/dm$^3$ at 25 C° has the value:

$$[\eta]_{0.1MHCl} = 30\ cm^3/g.$$

Example 2. The process is similar to the one described in Example 1, but into a retort 1.0 g of vinyl alcohol copolymer with N-vinylaminosuccinic acid (VA - VASA) (VASA content is 25 mol%, i.e. 52 wt%. 0.520g) and 20 $cm^3$ of distilled water are loaded, and then a mixer is turned on. After complete polymer solution, 0.650 g of 3.5-dimethyl-1-adamantylamine (DMAAM) is loaded. The initial equimolecular ratio of VASA:DMAAM is 1:1. The process is implemented at room temperature (20 - 25 C°) during 0.5 hour until complete homogenization of aquatic reaction medium. The product is produced by precipitation into acetone; it is filtrated, rinsed with acetone and diethyl ether and dried at 30 - 35 C°/6.65Pa. Based on the results of the elements analysis and potentiometric titration DMAAM content in a produced salt is 39.42 wt% which corresponds to 25 mol% of the VASA - DMAAM links (the links of 3.5-dimethyl-1-adamantylamine-ammonium salt of vinylaminosuccinic acid). Product output (VA - VASA - DMAAM) is 95%. Characterizing viscosity of a polymeric salt solution in HCl concentration c(HCl) 0.1mol/ $dm^3$ at 25 C° constitutes:

$$[\eta]_{0.1MHCl} = 14\ cm^3/g.$$

Example 3. The process is similar to the one described in Example 1, but into a reaction retort 0.991 g of the copolymer of N-vinylpyrrolidone with N-vinylaminosuccinic acid (VP - VASA) with the VASA links content 27 mol% and 50 $cm^3$ of distilled water are loaded. After polymer solution, 0.4 g of DMAAM is introduced. The reaction takes place at the temperature of 20 C° during 40 minutes. The product is produced by lyophilization (sublimation drying) of the solution, is irrigated by diethyl ether and dried at 30 - 35 C°/6.65 Pa. Produced salt contains 28.75 wt% of DMAAM corresponding to 27 mol% of the saline VASA - DMAAM links in the copolymer VP - VASA - DMAAM) Product output is 93%.

The value of characterizing viscosity of a polymeric salt solution in HCl concentration c(HCl) 0.1mol/$dm^3$ at 25 C° constitutes

$$[\eta]_{0.1MHCl} = 16\ cm^3/g.$$

All produced polymeric salts of 3.5-dimethyl-1-adamantylammonium are white amorphous powders, soluble in water, diluted acetic and hydrochloric acids, and dimethyl sulfoxide.

They are practically insoluble in 95% ethyl alcohol and diethyl ether. The values of characterizing viscosity of polymeric salt solutions in HCl water solution with the concentration c(HCl) 0.1 mol/dm3 at 25 C° are in the range of 10 - 30 $cm^3$/g.

Identification of produced compounds

In the IR spectrum of dry polymeric compounds being the invention essence, absorption bands at 1560 - 1610 $cm^{-1}$ are observed as corresponding to the valence vibrations of a C-O link of ionized group

$-COO^{-1}$; during formation of a salt link, absorption bands at 800 $cm^{-1}$ are detected characterizing the presence of $N^+H_3$ groups of ammonium salt, and there is no absorption band at 1720 $cm^{-1}$ (or the intensity of an initial polymer is reduced) corresponding to non-ionized -COOH groups in a polymer.

In the IR spectrum of produced salts there are also absorption bands at 1350 $cm^{-1}$ and 1000 $cm^{-1}$ characteristic for adamantan structure.

**Determination of biological properties of claimed compounds**

Immunostimulating activity. Determination of the effect of claimed compounds on functional activity of immunocomptent human cells.

Immunocompetent cells - natural killers (NK-cells) - relate to the system of natural human body resistance. Identification of the status of this immune system link gives important information about the state of the body immune status. To assess the state of this immune system link depending on the presence of studied compounds a functional

test was used to determine the NK-cells capacity to expose to lysis the target cells of tumor myeloblast culture K-562, the identification of which was implemented using a fluorescent tag. NK-cells activity was identified in accordance with the number of the target cells K-562 "killed" by them which were evaluated based on the intensity of fluorescent glowing in the area characteristic for killed cells and against control results (i.e. when NK-cells are absent).

The influence level of the studied drugs on NK-cells activity was assessed based on the value of the NK-cells activity ratio when treated with these medications to the activity of untreated NK-cells in the lysis process of target cells of the test culture, line K-562.

To select medication doses and concentrations while studying their activity, their minimal toxic dose against target cells and mononuclear cells of peripheral blood was preliminarily measured. This dose did not exceed 50 mkg/ml (as calculated per DMAAM).

In the result of studies it was found that with a low initial functional activity of immunocompetent cells of natural killers (NK-cells) of the mononuclear cells in peripheral blood the use of offered salts of 3.5-dimethyl-1-adamantylammonium leads to the increase of functional activity (Table 1). This defines a considerable immunostimulating effect of offered substances.

In addition immunostimulating effect mainly occurs in polymeric compounds (PVASA - DMAAM, VA - VASA - DMAAM, VP - VASA - DMAAM), and with an increase of drug concentration their stimulating impact on NK-cells activity enhances.

Table 1. Change of NK-cells activity over 3.5-dimethyl-1-adamantylamine.

| № | Compound | Relative value of change of NK-activity over DMAAM compounds with concentrations mcg/ml (as calculated per DMAAM): | | |
|---|---|---|---|---|
| | | 2 | 10 | 50 |
| 1 | HC - DMAAM | 1.0 | 1.2 | 1.6 |
| 2 | PVASA - DMAAM | 1.1 | 1.4 | 1.8 |
| 3 | VA - VASA - DMAAM | 1.1 | 1.4 | 1.8 |
| 4 | VP - VASA - DMAAM | 1.1 | 1.3 | 1.7 |

Interferoninducting activity of offered compounds

The capacity of interferon induction characterizes one of the levels of body shielding effect. Interferon induction by cells under the influence of some medications characterizes interferoninducing activity of these medications.

To measure the interferonogenic activity of the agents of claimed compounds, their activity in vitro was studied in relation to natural killer cells (NK-cells) of human (donors') peripheral blood with their spontaneous and induced interferonogenic activity.

The maximum used concentration of the compounds was 50mkg/ml (as calculated per DMAAM) which corresponds to the minimal toxic dose of medications for mononuclear cells of peripheral blood and test-culture. Interferonoinducing activity of the agents (Table 2) is expressed in international units (IU) of interferon antiviral activity as regards the virus of vesicular stomatitis. It was identified based on the calibration of the standard activity of test-interferon (IFN-$\alpha$).

Table 2. Interferon induction activity of mononuclear cells of peripheral blood (NK-cells) depending on the presence of DMAAM-compounds.

| № | Compound, interferon inducer | Interferon induction activity (IU/ml) of NK-cells over studied compounds with the said concentrations mcg/ml (as calculated per DMAAM): | | | |
|---|---|---|---|---|---|
| | | 0 | 2 | 10 | 50 |
| 1 | HC-DMAAM | - | 5.77 | 13.00 | 14.46 |
| 2 | PVASA - DMAAM | - | 10.25 | 24.23 | 25.52 |
| 3 | VA - VASA - DMAAM | - | 9.46 | 23.92 | 25.77 |
| 4 | VP - VASA - DMAAM | - | 8.45 | 23.80 | 25.20 |

(continued)

| № | Compound, interferon inducer | Interferon induction activity (IU/ml) of NK-cells over studied compounds with the said concentrations mcg/ml (as calculated per DMAAM): | | | |
|---|---|---|---|---|---|
| | | 0 | 2 | 10 | 50 |
| 5 | POLYREM | - | 8.12 | 20.50 | 21.68 |
| 6 | No inducer (spontaneous induction) | Less than 5 | | | |

The results of determinations presented in Table 2 show that offered compounds possess a rather effective level of interferon induction by mononuclear cells of peripheral blood. The interferon induction activity of the offered polymeric compounds PVASA-DMAAM, VA-VASA-DMAAM, VP-VASA-DMAAM 1.5 - 1.6 times exceeds the activity of a low-molecular HC-DMAAM hydrochloride.

It is important to notice that polymeric compounds DMAAM have greater interferonogenic activity than POLYREM (prototype) tested in the same conditions for comparison.

Thus, offered polymeric compounds have a noticeable immunostimulating and interferonogenic effect. They have potential to correct the activity of immunocompetent cells of peripheral blood mononuclear cells - natural killers, and of the interferoninducing activity of blood mononuclear cells, that is to improve the functional activity level of human immune system.

Determination of the toxic properties of compounds

The toxicity level of claimed compounds was assessed according to a general index of acute toxicity - average fatal dose of $LD_{50}$. The study was held with a single oral administration of the medication to white mice of both sexes, line "Balb", weight 18 - 20 g, and to white rats, line "Vistar", weight 130 - 250 g (female) and 225 - 370 g (male). The medications were administered to mice in the dose ranging from 25 to 1100 mg/kg, and to rats in the range of 480 - 2315 mg/kg.

It was determined that $LD_{50}$ values of claimed polymeric compounds DMAAM for mice are in the range from 445 to 806 mg/kg, and for rats - from 647 to 1180 mg/kg depending on the qualitative and quantitative composition of polymeric salts.

These results showed that claimed polymeric salts possessed lower toxicity as compared to memantin ($LD_{50}$ for mice 363 mg/kg) when agents with the equimolecular dose of 3.5-dimethyl-1-adamantylamine were administered to animals.

Offered salts in accordance with the toxicity indices determined for mice and rats refer to low-toxic compounds.

**Industrial use**

The information given in the sections "Summary of the invention" and "Determination of the biological properties of offered salts" proves that the synthesis of claimed salts is quite possible by the means described in the application. Produced compounds are expressly identified by IR analysis, elements analysis, and potentiometric titration methods. Offered compounds have high immunostimulating and interferonogenic activity. This activity is noticeably higher than the one of their prototype - polyrem.

In the process of comparative study, the inventors detected for the first time some immunostimulating and interferonogenic activity of a low-molecular compound from the offered compounds - hydrochloride 3.5-dimethyl-1-adamantylamine- which is considerably lower than the activity of offered polymers.

Thus, offered agents comply with all the requirements specified for an invention - they are new, unobvious for a specialist (have an invention level) and are exploitable.

**Claims**

**1.** Use of polymeric salts of 3.5-dimethyl-1-adamantylammonium and water-soluble carboxylated (co)polymers of the general formula

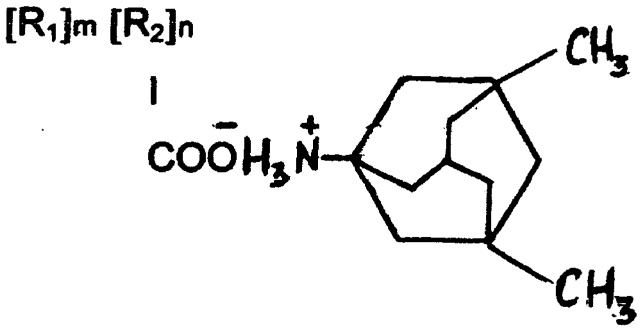

where m and n are mol%, m = 100 - n,
and carboxylated (co)polymers are selected from the group:
polyvinylaminosuccinic acid,
copolymer of vinyl alcohol and N-vinylaminosuccinic acid,
copolymer of N-vinylpyrrolidone and N-vinylaminosuccinic acid, as immunostimulating and interferonogenic agents.

**2.** Use as in claim 1, wherein polyvinylaminosuccinic acid (PVASA), where n = 1- 100mol%, and better n = 5 - 100mol% is selected as a carboxylated polymer.

**3.** Use as in claim 1, wherein a copolymer of vinyl alcohol and N-vinylaminosuccinic acid (VA)m - (VASA)n, where n = 1 - 99mol%, and better n = 5 - 50mol% is selected as a carboxylated polymer.

**4.** Use as in claim 1, wherein a copolymer of N-vinylpyrrolidone and N-vinylaminosuccinic acid (VP)m - (VASA)n, where n = 1 - 99mol%, and better n = 5 - 50mol% is selected as a carboxylated polymer.

**5.** A medicinal agent, wherein, any compound as in claims 1 - 4 is selected as an immunostimulating and interferonogenic substance.

## INTERNATIONAL SEARCH REPORT

International application No.
PCT/RU 2004/000242

A. CLASSIFICATION OF SUBJECT MATTER
A61K 31/785, A61P 31/00

According to International Patent Classification (IPC) or to both national classification and IPC

B. FIELDS SEARCHED

Minimum documentation searched (classification system followed by classification symbols)

A61K 31/785, 31/79, 31/13, 31/14, A61P 31/00, C07C 211/62, C08F 20/64, 8/44, 26/10

Documentation searched other than minimum documentation to the extent that such documents are included in the fields searched

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | RU 2071323 C1 (SANKT-PETERBURGSKY GOSUDARSTVENNY TEKHNOLOGICHESKY INSTITUT (TEKHNICHESKY UNIVERSITET) et al.) 10. 01. 1997 | 1-5 |
| A | RU 2185822 C2 (OBSCHESTVO S OGRANICHENNOI OTVETSTVENNOSTIJU "FARMAGEN") 27. 07. 2002 | 1-5 |
| A | RU 99113462 A (KORNING INKORPOREITED), 20.05.2001, (the abstract) | 1-5 |
| A | US 6613507 B1 (YU-AN CHANG) 02. 09. 2003 | 1-5 |
| A | Kozeletskaia K. N. et al. "Structure and antiviral activity adamantane-containing polymer preparation", Vopr Virusol. 2003 Sep-Oct; 48(5):19-26 (реферат) Найдено 2005-03-01. Found 2005-03-01. Found from data base MEDLINE. | 1-5 |
| A | DE 4014672 A (MUELLER WERNER E G PROF DR et al.) 14. 11. 1991 | 1-5 |
| A | RU 2219916 C2 (MERTS FARMA GMBKH UND KO, KGaA) 27. 12. 2003 | 1-5 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **23 February 2005 (23.02.2005)** | **17 March 2005 (17.03.2005)** |
| Name and mailing address of the ISA/ | Authorized officer |
| Facsimile No. | Telephone No. |

Form PCT/ISA/210 (second sheet) (July 1992)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- RU 2003000417 W
- WO 2005028528 A1
- WO 2005028528 A


### Non-patent literature cited in the description


- **M.D. MASHKOVSKI.** Medicinal Agents. *New Wave,* 2002
- **YU.F. KRYLOV.** Encyclopedia of Drugs/Register of Medicinal Agents of Russia. 2000
- **M.D. MASHKOVSKI.** Medicinal Agents. *New Wave,* 2002, vol. 1, 143
- *NEW WAVE,* vol. 1, 142
- *NEW WAVE,* vol. 2, 325
- *NEW WAVE,* 142, 143, 325
- **V.A. SNEZHKO et al.** *Antibiotics,* 1972, vol. 17 (1), 48-52
- **E.F. RAZVODOVSKI.** *Progress of Polymer Chemistry and Physics, M, Chemistry,* 1973, 305
- **F.I. ERSHOV.** *Antiviral Medications, M, Medicine,* 1998, 141-142
- **A.F. NIKOLAEV et al.** *The Journal of Applied Chemistry,* 1970, vol. 43 (6), 1339-1343
- **A.F. NIKOLAEV et al.** *High-molecular Compounds,* 1972, vol. 14A (11), 2368-2370